Europäisches Patentamt

European Patent Office

Office européen des brevets

(11) Publication number: **0 448 072 A2**

# EUROPEAN PATENT APPLICATION

(21) Application number: 91104327.1

(22) Date of filing: 20.03.91

(51) Int. Cl.5: **G01N 33/72**, C12Q 1/26

(30) Priority: 20.03.90 JP 70388/90

(43) Date of publication of application:
**25.09.91 Bulletin 91/39**

(84) Designated Contracting States:
**BE CH DE DK ES FR GB IT LI NL SE**

(71) Applicant: THE GREEN CROSS CORPORATION
3-3, Imabashi 1-chome Chuo-ku
Osaka-shi Osaka(JP)

(72) Inventor: Nagatomo, Midori, c/o The Green
Cross Corp.
Miyakojima Fact., 5-44, Miyakojimanakadori
3-chome
Miyakojima-ku, Osaka-shi, Osaka(JP)
Inventor: Shimizu, Toru, c/o The Green Cross
Corp.
Miyakojima Fact., 5-44, Miyakojimanakadori
3-chome
Miyakojima-ku, Osaka-shi, Osaka(JP)
Inventor: Toyoda, Takayuki, c/o The Green
Cross Corp.
Miyakojima Fact., 5-44, Miyakojimanakadori
3-chome
Miyakojima-ku, Osaka-shi, Osaka(JP)

(74) Representative: Hansen, Bernd, Dr.
Dipl.-Chem. et al
Hoffmann, Eitle & Partner Patent- und
Rechtsanwälte Arabellastrasse 4 Postfach
81 04 20
W-8000 München 81(DE)

(54) Process for assaying free hemoglobin and reagent kit for assaying free hemoglobin.

(57) A process for assaying free hemoglobin which comprises contacting a free hemoglobin-containing specimen with a haptoglobin-immobilized solid phase where human haptoglobin is bound to a solid phase, to thereby bind free hemoglobin to the human haptoglobin on the solid phase, treating the free hemoglobin with a peroxide reagent solution and a coloring reagent solution and then measuring the optical change induced in said solution, as well as a reagent kit to be used in the above assay process.

EP 0 448 072 A2

FIELD OF THE INVENTION

This invention relates to a process for assaying free hemoglobin and a reagent kit for assaying free hemoglobin. More particularly, it relates to a process whereby free hemoglobin can be easily and rapidly assayed, e.g., for a clinical purpose as well as a reagent kit for assaying free hemoglobin.

BACKGROUND OF THE INVENTION

Massive blood transfusion, extracorporeal circulation, hemodialysis and organ perfusion frequently cause serious hemolysis which might be accompanied by various complicating diseases such as hemolytic renal disorder. It is said that free hemoglobin (not bound to haptoglobin) causes these complicating diseases. When hemoglobin contained in plasma or serum is to be assayed for a clinical purpose, it is therefore required to separate the hemoglobin bound to haptoglobin from free or unbound hemoglobin.

Thus the assay of free hemoglobin contained in, for example, plasma or serum, is important from a clinical viewpoint. Known methods for assaying free hemoglobin include cellulose acetate electrophoresis, an abbreviated fractionation method and a free hemoglobin adsorption method (using a column).

The above-mentioned cellulose acetate electrophoresis procedure for assaying free hemoglobin may be performed in the following manner. First, the total hemoglobin in a specimen (plasma or serum) is determined by the cyanmethemoglobin method (Crosby, W.H. et al., Blood, 12, 1132-1136 (1966)). Then said specimen is divided into free hemoglobin, haptoglobin/hemoglobin complex and methemoalbumin (hemoglobin bound to albumin) fractions by cellulose acetate electrophoresis. After staining the hemoglobin through a peroxidase reaction, the degree of staining of each fraction is scanned with a densitometer. Thus the ratio of the free hemoglobin to the total hemoglobin (free hemoglobin + haptoglobin/hemoglobin complex + methemoalbumin) is determined. The ratio thus determined is multiplied by the total hemoglobin content, which has been preliminarily determined by the cyanmethemoglobin method, to thereby calculate the content of the free hemoglobin in the specimen.

The above-mentioned abbreviated fractionation method for assaying free hemoglobin may be performed in the following manner. The total hemoglobin is determined by the cyanmethemoglobin method while the total haptoglobin is determined by single immunodiffusion method (Mancini method; Mancini, G. et al., Immunochemistry, 2, 233-234 (1965)). Then, the free hemoglobin is calculated with the use of the haptoglobin/hemoglobin binding ratio thus determined. In the determination of the total haptoglobin by the single immunodiffusion method, the serum type of haptoglobin is preliminarily identified by starch gel electrophoresis, immunoelectrophoresis or acrylamide gel electrophoresis and a standard curve of the corresponding serum type is employed. On the other hand, when a standard curve formed with the use of pooled serum is to be used, it is required to multiply the data obtained with the use of the standard curve by the coefficient of the corresponding serum type of haptoglobin to thereby provide the data used in the determination.

The above-described hemoglobin adsorption method (JP-A-54-150885; the term "JP-A" used herein means an unexamined published Japanese patent application) takes advantage of the property that haptoglobin adsorbs hemoglobin. The determination of free hemoglobin by this method may be performed in the following manner. A definite amount of a specimen (plasma or serum) is poured into a cylindrical column uniformly packed with haptoglobin-immobilized Sepharose. After washing with approximately 3 to 10 times as much physiological saline, the length of the part colored in red with the free hemoglobin adsorbed by the haptoglobin-immobilized Sepharose is measured. Thus the content of the free hemoglobin is determined.

The above-mentioned cellulose acetate membrane electrophoresis is disadvantageous in that the operation is complicated and requires a long time, and since the content of the free hemoglobin is indirectly determined by multiplying the total hemoglobin content by the fractionation ratio. Furthermore, a content of free hemoglobin lower than the determination limit for total hemoglobin, or the detectable limit, by the cellulose acetate electrophoresis cannot be determined by this method.

According to the above-mentioned abbreviated fractionation method, wherein the content of free hemoglobin is indirectly determined from the total hemoglobin content and the total haptoglobin content, it is impossible to determine the content of free hemoglobin lower than the determination limit for total hemoglobin. Furthermore, this method suffers from a problem that hemoglobin bound to haptoglobin in an amount lower than the determination limit of the total haptoglobin is consequently calculated as free hemoglobin.

The above-mentioned free hemoglobin adsorption method, which comprises pouring a definite amount of a specimen (serum or plasma) into a column packed with haptoglobin-immobilized Sepharose and

2

measuring the length of a layer of the column colored by the free hemoglobin adsorbed by the haptoglobin-immobilized Sepharose so as to determine the content of the free hemoglobin in the specimen, suffers from such problems that the length of the colored layer might vary depending on the uniformity of the haptoglobin-immobilized Sepharose in the column, the method for pouring the specimen and/or the employed washing method; that the end point of the colored layer is unclear; and that the quantitative accuracy and reproducibility are limited.

Additionally, each of the known assay methods as described above suffers from such disadvantages that the preparation of the reagents to be used and the assay operation are troublesome, and that a long time is required for obtaining the result. Thus, they are scarcely applicable to a qualitative analysis whereby the presence of free hemoglobin in a specimen should be rapidly examined.

## SUMMARY OF THE INVENTION

The present invention has been achieved in order to overcome the above-described disadvantages of the prior art. Therefore, it is an object of the present invention to provide a process for assaying free hemoglobin, whereby free hemoglobin contained in a specimen (for example, serum, plasma, urine, feces) can be rapidly and specifically determined (quantitatively and qualitatively) through a simple operation, as well as a reagent kit for assaying free hemoglobin.

The process of the the present invention for assaying free hemoglobin comprises contacting a free hemoglobin-containing specimen with a haptoglobin-immobilized solid phase, wherein human haptoglobin is bound to a solid phase, to thereby bind the free hemoglobin in the specimen to the human haptoglobin on the solid phase, treating the hemoglobin bound to the haptoglobin with a peroxide reagent solution and a coloring reagent solution and then measuring the optical change thus induced in said solution. The reagent kit of the present invention for assaying free hemoglobin, which is to be used in the above-described assay process, comprises a haptoglobin-immobilized solid phase wherein human haptoglobin is bound to a solid phase, a peroxide reagent and a coloring reagent.

## BRIEF DESCRIPTION OF THE DRAWING

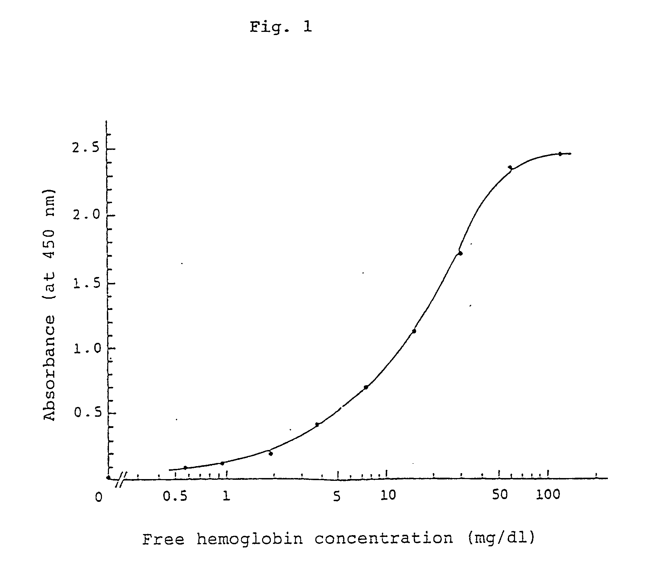

Fig. 1 is a graph which shows a relationship of the concentration of free hemoglobin and absorbance in the assay of free hemoglobin by the process of the present invention with the use of a haptoglobin-immobilized membrane.

## DETAILED DESCRIPTION OF THE INVENTION

According to the present invention, the haptoglobin of the haptoglobin-immobilized solid phase, wherein human haptoglobin is bound to a solid phase, is specifically bound to free hemoglobin in the free hemoglobin-containing specimen. Thus the free hemoglobin can be selectively and irreversibly bound to the haptoglobin fixed on said solid phase through noncovalent bond between tyrosine residues of $\beta$ chain of haptoglobin and $\alpha$ chain of hemoglobin. Since hemoglobin has a peroxidase-like activity, a coloring pigment may be formed by treating said solid phase on which the free hemoglobin is fixed with a peroxide reagent and a color reagent. Therefore, the presence of free hemoglobin can be examined by measuring the optical change thus induced by the free hemoglobin. The optical change depends on the amount of the free hemoglobin bound to the haptoglobin-immobilized solid phase. Further, the amount of the free hemoglobin bound to the haptoglobin-immobilized solid phase depends on the content of the free hemoglobin in the specimen. Therefore the free hemoglobin contained in the specimen can be determined by measuring the optical change which thus correlates to the free hemoglobin content in the specimen.

The human haptoglobin to be used in the preparation of the haptoglobin-immobilized solid phase employed in the present invention may be human haptoglobin prepared from human plasma by a known method (Rinsho (Clinical) Haptoglobin, p. 223, T. Oshiro, Nagai Shoten (1987)), which may be further purified by affinity chromatography using anti-human hemoglobin antibody so as to remove a hemoglobin/haptoglobin complex (adsorbed fraction) from human haptoglobin (unadsorbed fraction). Specifically, the affinity chromatography can be conducted by immobilizing anti-human hemoglobin antibody on CNBr-activated Sepharose 4B (Pharmacia) in accordance with the method provided by the manufacturer, packing the immobilized product into a column followed by equilibration with an appropriate buffer such as phosphate buffer, applying human haptoglobin equilibrated with the same buffer onto the column and collecting a fraction which passes through the column. The haptoglobin-immobilized solid phase may be prepared by, for example, immobilizing the human haptoglobin obtained in the above-mentioned manner on a solid phase by a known method, for example, by adsorption, or by the bromocyanogen method (Koso Meneki Sokuteiho (Enzyme Immunoassay), 3 ed., p. 395, Igaku Shoin (1987)). The immobilization by adsorption is conducted by dissolving the human haptoglobin in an appropriate buffer solution (e.g., phosphate buffer (pH 7.0), carbonate buffer (pH 9.6), etc.), distributing the resulting solution onto an ELISA plate and allowing the plate to stand overnight at $4°C$ to thereby immobiliging the haptoglobin through the hydropholic bond (adsorption). In accordance with the bromocyanogen method, the human haptoglobin is immobilized by suspending a CNBr-activated agarose resin into a buffer solution (e.g. phosphate buffer (pH 8.0)) containing the haptoglobin or distributing the buffer solution onto an ELISA plate and allowing the resulting suspension or solution to stand overnight at $4°C$ with shaking. Alternatively, immobilization of the haptoglobin can carried out by coupling of a carboxyl group or an amino group in the haptoglobin molecule and free amino groups or carboxyl groups which are bound to an ELISA plate or a nitrocellulose membrane in the presence of carbodiimide.

As the solid phase material on which the human haptoglobin is to be immobilized, any one conventionally employed in, for example, enzyme immunoassay may be used. Examples thereof include polysaccharides such as agarose and Sepharose, glass, ceramics and plastics. Although the shape of these materials is not particularly restricted, they are preferably in the form of, for example, a plastic membrane (sheet or film) such as nitrocellulose membrane, nylon membrane or cellulose membrane, a plastic tube or a plastic ball, by taking the convenience of the assay operation into consideration. The solid phase may be in the form of a stick wherein a haptoglobin-immobilized membrane prepared by immobilizing haptoglobin on the above-mentioned plastic membrane is fixed to one end of a stick-shaped substrate made of, for example, plastic. The amount of the human haptoglobin immobilized on the solid phase is not particularly restricted. It may be appropriately controlled depending on, for example, the desired sensitivity by controlling the concentration of bromocyanogen group to be introduced into the solid phase, or the concentration of the human haptoglobin. The concentration of the human haptoglobin solution to be used in the immobilization may range from 5 $\mu$g/ml to 5 mg/ml.

The peroxidase-like activity of the free hemoglobin bound to the haptoglobin-immobilized solid phase may be determined by a method which is substantially the same as the enzyme immunoassay process wherein peroxidase is employed as a labeled enzyme. Therefore the peroxide reagent and the coloring reagent to be used in the determination of the peroxidase-like activity of the free hemoglobin may be selected from among those employed in enzyme immunoassay. More specifically, examples of the peroxide reagent include hydrogen peroxide, strontium peroxide, barium peroxide, cumene hydroperoxide and dimethylhexane peroxide. Among these compounds, hydrogen peroxide is particularly suitable. Examples of the coloring reagent include 3,3',5,5'-tetramethylbenzidine, 3,3',5,5'-tetramethylbenzidine dihydrochloride, o-phenylenediamine, benzidine, o-tridine, o-dianisidine, diphenylamine, guaiac resin, amidopyrine, hemogreen and phenolphthalin. Among these compounds, 3,3',5,5'-tetramethylbenzidine and o-phenylenediamine are particularly preferred.

Now, an example of the assay process of the present invention will be described in detail. First, purified human haptoglobin is immobilized on an appropriate solid phase material by a method selected from among the above-described ones. Thus, a haptoglobin-immobilized solid phase is obtained. Then the haptoglobin-immobilized solid phase is immersed for about 0.5 to 1 minute in a free hemoglobin-containing specimen (for example, plasma, serum, urine), which has been preliminarily diluted with, for example, purified water in such a manner as to give an appropriate concentration, if required. Thus, the free hemoglobin contained in the specimen is exclusively and specifically bound to the haptoglobin on the haptoglobin-immobilized solid phase. Next, the haptoglobin-immobilized solid phase is washed with, for example, purified water, if required. The washing may be performed in an appropriate manner selected depending on the form of the haptoglobin-immobilized solid phase. When the haptoglobin-immobilized solid phase is in the form of a membrane, for example, it may be immersed or shaken in purified water. When the haptoglobin-immobilized solid phase is in the form of particles, the washing supernatant may be sucked off. By this washing operation, a haptoglobin/hemoglobin complex, which might be nonspecifically bound (adsorbed) to the haptoglobin-immobilized solid phase, is removed, while the hemoglobin specifically bound to the haptoglobin exclusively remains on the haptoglobin-immobilized solid phase without being removed by washing. The hemoglobin bound haptoglobin-immobilized solid phase is immersed in a reagent solution containing peroxide reagent and coloring reagent for 2 to 5 minutes. The reaction is terminated by adding a strong acid such as a sulfuric acid or a hydrochloric acid and the optical change thus induced is measured. Thus the peroxidase-like activity of the free hemoglobin is determined and the free hemoglobin contained in the specimen is assayed. The optical change may be measured by, for example, colorimetry, fluorometry, or chemical emission analysis. It is preferable to measure the optical change by absorptiometry, since this method can be easily performed and is excellent in performance.

In the case of using 3, 3', 5, 5'-tetramethylbenzidine as a coloring reagent, the absorbance is measured at 450 nm. In the case of using o-phenylenediamine, the absorbance is measured at 492 nm.

The free hemoglobin contained in the specimen may be determined in the following manner. A calibration curve showing a relationship between free hemoglobin concentration and optical change is formed by repeating the above procedure except that the free hemoglobin-containing specimen is replaced with sample solutions obtained by appropriately diluting a standard free hemoglobin solution of a known concentration. Then the optical change determined with the use of the free hemoglobin specimen is compared with the calibration curve to thereby determine the free hemoglobin content in the specimen. The free hemoglobin may be qualitatively examined by observing the coloration or discoloration of the reaction mixture with, for example, the naked eye.

In the above assay process, the concentration of the free hemoglobin in the specimen is adjusted to at least 1 mg/dl. The detectable limit of the free hemoglobin is about 0.3 to 0.5 mg/dl as can be seen from Fig. 1. When the content of free hemoglobin is to be determined, said concentration may be adjusted to 1 to 100 mg/dl, preferably 5 to 50 mg/dl. The peroxide concentration of the peroxide reagent solution to be used may be usually adjusted to 0.01 to 1.0 w/v %, preferably 0.05 to 0.1 w/v %, in the case of, for example, an aqueous solution of hydrogen peroxide. The coloring reagent concentration of the coloring reagent solution to be used may be usually adjusted to 0.1 to 4 mM, preferably 1.0 to 3.0 mM, in the case of, for example, 3,3',5,5'-tetramethylbenzidine. The peroxide reagent solution and the coloring reagent solution are usually used in the form of an equivalent mixture. They are mixed just before use. The above-mentioned determination may be usually carried out at room temperature and completed within 10 to 15 minutes.

The reagent kit for assaying free hemoglobin of the present invention at least comprises a haptoglobin-immobilized solid phase, wherein human haptoglobin is bound to a solid phase, a peroxide reagent and a coloring reagent. The haptoglobin-immobilized solid phase is usually provided in a dry state through, for example, lyophilizing in order to improve the shelf life and stability thereof. The peroxide reagent and the coloring reagent are usually provided in the form of an aqueous solution which optionally contains a stabilizer, for example, phosphate, uric acid in the case of hydrogen peroxide or metal masking agent such as ethylenediaminetetraacetic acid (EDTA) in the case of o-phenylenediamine. The reagent kit of the present invention may further comprise a solid phase on which no human haptoglobin is immobilized. Furthermore, it may comprise a standard free hemoglobin solution to be used for the formation of a calibration curve.

According to the process for assaying free hemoglobin and the reagent kit for assaying free hemoglobin of the present invention, wherein a haptoglobin-immobilized solid phase capable of specifically binding to free hemoglobin is used, free hemoglobin contained in a specimen (for example, serum, plasma, urine, feces) can be directly assayed at a high sensitivity. In this process, the assay is performed by taking advantage of the peroxidase-like activity of free hemoglobin, which brings about further advantages such that the employed reagents can be easily prepared, the assay operation can be conveniently effected and the assay can be completed within a short period of time. Therefore, the process and reagent kit of the present invention can be widely applied to the quantitative and qualitative determination of free hemoglobin.

To further illustrate the present invention, the following non-limiting Preparation Examples and Examples will be given.

Preparation Example 1

Preparation of haptoglobin-immobilized solid membrane

Purified human haptoglobin obtained from a normal human plasma pool free from hemoglobin (mfd. by The Green Cross Co.) was dissolved in a phosphate-buffered physiological saline (PBS) so as to give a human haptoglobin concentration of 1 mg/ml. Thus a solution of human haptoglobin was obtained.

Then, a nitrocellulose membrane (45 x 50 x 0.05 mm) was immersed in the above human haptoglobin solution at room temperature for approximately 16 hours. After the membrane was placed lightly on a filter paper to remove water, it was then immersed in PBS containing 0.25 % of bovine serum albumin at room temperature for approximately 16 hours. Then the nitrocellulose membrane was dried under reduced pressure and cut into pieces (15 x 5 mm) to thereby give a haptoglobin-immobilized membrane.

Preparation Example 2

Preparation of standard free hemoglobin solution

Human hemoglobin prepared by a method reported by Williams [Anal. Biochem., 54, 137 - 145 (1973)] was subjected to affinity chromatography using anti-human haptoglobin antibody and the unadsorbed fraction was collected while the adsorbed fraction (hemoglobin/haptoglobin complex) was removed. The hemoglobin contained in the collected unadsorbed fraction was determined by cyanmethemoglobin method. Thus a standard free hemoglobin solution was obtained.

Example 1

Determination (qualitative) of free hemoglobin in serum

Using the haptoglobin-immobilized membrane prepared in the above Preparation Example 1 (hereinafter referred to as the membrane a), free hemoglobin contained in human serum (each of 37 specimens) was qualitatively determined in the following manner.

The membrane a and another membrane employed as a control, on which no haptoglobin was fixed, (hereinafter referred to as the membrane b), which was prepared in the same manner as in Preparation Example 1 except that the membrane was not treated with haptoglobin, were simultaneously immersed in a tube containing a specimen (human serum). After incubating at room temperature for 1 minute, the membranes a and b were taken out and sealed in a container containing approximately 30 ml of water for injection. Then, the container was vigorously shaken vertically (about 20 times) to thereby wash the membranes. This washing procedure was repeated while exchanging the water (thrice in total). Then the membranes a and b were taken out from the container and placed lightly on a filter paper for removing water.

Separately, a 0.1 w/v % aqueous solution of hydrogen peroxide and 2 mM solution of 3,3',5,5'-tetramethylbenzidine were mixed at a ratio of 1 : 1 by volume. 1 ml portions of the solution thus obtained were introduced into two tubes. Then the above membranes a and b were respectively immersed in these tubes. After incubating at room temperature for 5 minutes, the coloration of the membrane a was observed while using the membrane b as a control. A specimen showing coloration was referred to as positive ( + ) for hemoglobin, while one showing no coloration was referred to as negative (-) therefor.

For comparison, the free hemoglobin contents of the same serum specimens were determined by the free hemoglobin fractionation assay method (Rinsho Haptoglobin, T. Oshiro, Nagai Shoten, p. 28) and the correlation of the obtained data with those obtained by the above assay process of the present invention was examined. Table 1 shows the results. In Table 1, " + " means a specimen showing free hemoglobin while "-" shows one showing no free hemoglobin.

As shown in Table 1, the data achieved by the assay process of the present invention agrees well with those of the fractionation assay method.

Table 1

| Invention assay | | Fractionation assay | |
|---|---|---|---|
| + | 24 | + | 24 |
| | | - | 0 |
| - | 13 | + | 1 |
| | | - | 12 |

Example 2

Formation of calibration curve with the use of standard free hemoglobin

The standard free hemoglobin solution prepared in Preparation Example 2 was diluted with purified water to thereby give a free hemoglobin concentration of 0.5 to 100 mg/dl per ml. Thus standard free hemoglobin sample solutions of various concentrations were obtained.

Using each standard free hemoglobin solution thus obtained, the procedure of Example 1 was repeated to induce the coloration. After ceasing the reaction by adding 0.1 ml of 2 N sulfuric acid, the absorbance of the sample was measured at 450 nm. The measurement of the absorbance of each standard sample was repeated thrice and the average value was calculated. The average absorbance was plotted against free hemoglobin concentration to form a calibration curve which is shown in Fig. 1.

Example 3

Using 24 human serum specimens, the free hemoglobin contents of which had been preliminarily determined by the fractionation assay method as mentioned in Example 1, the procedure of Example 1 was repeated. Then the reaction was ceased by adding 0.1 ml of 2 N sulfuric acid to each specimen and the absorbance thereof was measured at 450 nm.

The absorbances thus measured were compared with the calibration curve obtained in the above Example 2 to determine the free hemoglobin content of each human serum specimen. Table 2 shows the results.

## Table 2

| | Free hemoglobin content (mg/dl) | |
|---|---|---|
| Specimen No. | Fractionation assay | Invention assay |
| 1 | 22.5 | 18.5 |
| 2 | 83.6 | 71.8 |
| 3 | 36.4 | 21.7 |
| 4 | 61.6 | 53.6 |
| 5 | 41.9 | 40.6 |
| 6 | 53.1 | 63.5 |
| 7 | 29.9 | 24.0 |
| 8 | 33.2 | 31.1 |
| 9 | 47.9 | 37.8 |
| 10 | 74.9 | 72.6 |
| 11 | 41.1 | 47.7 |
| 12 | 121.3 | 119.4 |
| 13 | 15.6 | 13.9 |
| 14 | 70.2 | 64.6 |
| 15 | 50.8 | 45.8 |
| 16 | 87.3 | 75.3 |
| 17 | 105.4 | 96.4 |
| 18 | 65.4 | 57.9 |
| 19 | 213.7 | 190.0 |
| 20 | 97.5 | 83.6 |
| 21 | 232.7 | 202.5 |
| 22 | 209.7 | 217.6 |
| 23 | 94.8 | 89.7 |
| 24 | 61.3 | 56.3 |

As shown in Table 2 the data obtained by the assay process of the present invention well correlates to those obtained by the fractionation assay method.

While the invention has been described in detail and with reference to specific embodiments thereof, it will be apparent to one skilled in the art that various changes and modifications can be made therein without departing from the spirit and scope thereof.

**Claims**

1. A process for assaying free hemoglobin which comprises contacting a free hemoglobin-containing specimen with a haptoglobin-immobilized solid phase where human haptoglobin is bound to a solid phase, to thereby bind free hemoglobin to the human haptoglobin on the solid phase, treating the free hemoglobin with a peroxide reagent solution and a coloring reagent solution and then measuring the optical change induced in said solution.

2. A reagent kit for assaying free hemoglobin which comprises a haptoglobin-immobilized solid phase where human haptoglobin is immobilized on a solid phase, a peroxide reagent and a coloring reagent.

3. A reagent kit for assaying free hemoglobin as claimed in Claim 2, wherein the solid phase used in the haptoglobin-immobilized solid phase is a plastic membrane, a plastic ball or a plastic tube.

4. The process of claim 1 which is carried out as a quantitative process.

5. The process of claim 1 carried out as qualitative process.

6. The process of claim 4, wherein a standard calibration curve is employed.

7. The process of claim 1, wherein the peroxide reagent solution and the coloring reagent solution are combined and the combined solution is used to treat the free hemoglobin.

8. The process of claim 1, wherein the peroxide reagent is selected from the group consisting of hydrogen peroxide, strontium peroxide, barium peroxide, cumene hydroperoxide and dimethylhexane peroxide.

9. The process of claim 1, wherein the peroxide reagent is hydrogen peroxide.

10. The process of claim 1, wherein the coloring reagent is selected from the group consisting of 3, 3', 5, 5'-tetramethylbenzidine, 3, 3', 5, 5'-tetramethylbenzidine dihydrochloride, o-phenylenediamine, benzidine, o-tridine, o-dianisidine, dipheylamine, guaiac resin, amidopyrine, hemogreen and phenolphtalin.

11. The process of claim 1, wherein the coloring reagent is 3, 3', 5, 5'-tetramethylbenzidine or o-phenylenediamine.

12. The process of claim 1, wherein the solid phase used in the haptoglobin-immobilized solid phase is a plastic membrane, a plastic ball or a plastic tube.

13. The reagent kit of claim 2, wherein the peroxide reagent is selected from the group consisting of hydrogen peroxide, strontium peroxide, barium peroxide, cumene hydroperoxide and dimethylhexane peroxide.

14. The reagent kit of claim 2, wherein the peroxide reagent is hydrogen peroxide.

15. The reagent kit of claim 2, wherein the coloring reagent is selected from the group consisting of 3, 3', 5, 5'-tetramethylbenzidine,3, 3', 5, 5'-tetramethylbenzidine dihydrochloride, o-phenylenediamine, benzidine, o-tridine, o-dianisidine, dipheylamine, guaiac resin, amidopyrine, hemogreen and phenolphtalin.

16. The reagent kit of claim 2, wherein the coloring reagent is 3, 3', 5, 5'-tetramethylbenzidine or o-phenylenediamine.

Fig. 1

Free hemoglobin concentration (mg/dl)